# EUROPEAN PATENT APPLICATION

(11) **EP 2 216 647 A1**
(43) Date of publication of application: **11.08.2010**
(21) Application number: 08854654.4
(22) Date of filing: 26.11.2008
(51) Int. Cl.: G01N 27/333, B01J 13/04, G01N 27/416, G01N 33/544

(54) **METHOD OF FORMING ARTIFICIAL LIPID FILM OVER MINUTE HOLE IN SUBSTRATE AND SUBSTRATE HAVING THE ARTIFICIAL LIPID FILM**

(30) Priority: 29.11.2007 JP 2007309059
(71) Applicant: The University of Tokyo, Bunkyo-Ku Tokyo 113-8654 (JP)
(72) Inventor: TAKEUCHI, Shoji, Tokyo 113-8654 (JP); KURIBAYASHI, Kaori, Tokyo 113-8654 (JP)
(74) Representative: Hoarton, Lloyd Douglas Charles
(86) International application number: PCT/JP2008/071369
(87) International publication number: WO 2009/069609

(57) **Abstract**

There is provided a method of preparing organic-solvent-free artificial lipid membranes formed over microapertures of a substrate and a substrate for holding the artificial lipid membranes. The method of preparing artificial lipid membranes over microapertures of a substrate includes forming a lipid pattern 5 on the perimeter of microapertures 4 formed in a substrate 1, swelling the lipid pattern 5 by an electroformation method, fusing the swelled lipid pattern, and further swelling the fused lipid pattern for each microaperture to prepare dome-shaped organic-solvent-free lipid membranes 7.

## Description

### Technical Field

The present invention relates to a method of preparing artificial lipid membranes over microapertures of a substrate and the substrate for holding the artificial lipid membranes.

### Background of Art

Artificial planar lipid membranes have been widely used as a powerful tool to analyze membrane proteins. The membrane proteins are reconstructed into the artificially formed planar lipid bilayer (see Non-Patent Document 1 below). The common methods to prepare the artificial planar lipid membranes are the painting method and Langmuir-Blodgett method (LB method) (see Non-Patent Document 2 below). They use lipids mixed with organic solvents such as decane (C₁₀H₂₂). However, it is difficult to evaporate the organic solvent completely from the prepared lipid membranes. Since the organic solvents may damage the protein, the lipid membrane with the solvent remained is not an ideal environment for the membrane protein analyses. Therefore, the organic-solvent-free lipid membranes are highly required. Recently, several methods have been reported for preparing organic-solvent-free lipid bilayers using giant unilamellar liposomes (see Non-Patent Documents 3 and 4 below). The giant liposomes are positioned on microapertures by suction or electrophoresis and fused with a substrate to form bilayers. The electroformation method (see Non-Patent Document 5 below) has been used to prepare giant unilamellar liposomes. The inventors have prepared monodisperse giant liposomes from patterned lipid films using the electroformation method (see Non-Patent Document 6 below).
Non-Patent Document 1: C. Miller, Ion Channel Reconstitution, Plenum Pub, Corp, New York, 1986.
Non-Patent Document 2: M. Montal, P. Mueller, "Formation of Bimolecular Membranes from Lipid Monolayers and a Study of their Electrical Properties", Proc. Natl. Acad. Sci., vol. 69, pp. 3561-3566, 1972.
Non-Patent Document 3: C. Schmidt, M. Mayer, H. Vogel, "A Chip-Based Biosensor for the Functional Analysis of Single Ion Channels", Angew. Chem. Int. Ed, vol. 39, pp. 3137-3140, 2000.
Non-Patent Document 4: M. Sondermann, M. George, N. Fertig, J. Behrends, "High-Resolution Electrophysiology on a Chip: Transient Dynamics of Alamethicin Channel Formation", Biochimica et Biophysica Acta, vol. 1758, pp. 545-551, 2006.
Non-Patent Document 5: M. Angelova, D. Dimitrov, "Liposome electroformation", Faraday Discuss. Chem. Soc., vol. 81, pp. 303-311 and 345, 1986.
Non-Patent Document 6: K. Kuribayashi, S. Takeuchi, "Formation of Monodisperse Giant Liposomes using Micro-Patterned Lipid Films", in The Proceedings of microTAS '05 Conference, Boston, October 9-13, 2005, pp. 1455-1457.
Non-Patent Document 7: K. Kuribayashi, S. Takeuchi, "Sequential Parylene Lift-Off Process for Selective Patterning of Biological Materials", in The Proceedings of MEMS '07 Conference, Tokyo, January 21-25, 2007, pp. 10-15.

### SUMMARY OF THE INVENTION

Accordingly, the present invention proposes a method of preparing organic-solvent-free artificial lipid membranes over microapertures of a substrate and the substrate for holding the artificial lipid membranes.

An advantage of the present invention is that the organic-solvent-free artificial lipid membranes can be prepared directly over the microapertures of a microaperture array without relying on suction or electrophoresis. In addition, the organic-solvent-free artificial lipid membranes in an array can be easily prepared.

In view of above, the present invention is directed to provide the method of preparing artificial lipid membranes over the microapertures of the substrate and the substrate for holding the artificial lipid membranes.

In order to achieve the object described above, the present invention provides the following:
[1] A method of preparing artificial lipid membranes over microapertures of a substrate, comprising the steps of: forming a lipid pattern on the perimeter of the microapertures formed in the substrate; and swelling the lipid pattern by an electroformation method, fusing the swelled lipid pattern, and further swelling the fused lipid pattern for each microaperture to prepare dome-shaped organic-solvent-free lipid membranes.

[2] The method of preparing artificial lipid membranes over microapertures of a substrate according to [1], wherein the substrate is made of a Parylene film having an electrode film deposited thereon.

[3] The method of preparing artificial lipid membranes over microapertures of a substrate according to [2], wherein the electrode film is a Cr/Au electrode film.

[4] The method of preparing artificial lipid membranes over microapertures of a substrate according to [2], wherein the lipid pattern on the perimeter of the microapertures is formed by a Parylene lift-off technique.

[5] The method of preparing artificial lipid membranes over microapertures of a substrate according to [4], wherein the lipid pattern on the perimeter of the microapertures is formed by depositing a first Parylene film on a bottom substrate, depositing the Cr/Au electrode film thereon, etching the first Parylene film and the Cr/Au electrode by lithography, then forming an intermediate layer on the Cr/Au electrode film, depositing a second Parylene film on the intermediate layer, etching the second Parylene film and the intermediate layer by the lithography, applying a lipid solution thereon, peeling off the second Parylene film and the bottom substrate, and storing in a vacuum chamber for a few hours.

[6] The method of preparing artificial lipid membranes over microapertures of a substrate according to [5], wherein the intermediate layer is ODT.

[7] The method of preparing artificial lipid membranes over microapertures of a substrate according to [1], wherein the dome-shaped organic-solvent-free lipid membranes are prepared by disposing the substrate with the lipid pattern formed on the perimeter of the microapertures in a chamber containing a buffer introduced therein, and using the electroformation method.

[8] A substrate for holding artificial lipid membranes comprising dome-shaped organic-solvent-free artificial lipid membranes formed over microapertures of a substrate having a film with an electrode film deposited thereon, that is peeled off easily.

[9] The substrate for holding artificial lipid membranes according to [8], wherein the film that is peeled off easily is made of a Parylene film.

### Brief Description of Drawings

Fig. 1 is a perspective view showing a formation process of an artificial lipid membrane array having dome-shaped artificial lipid membranes over microapertures illustrating an embodiment of the present invention;
Fig. 2 is a sectional view showing a formation process of preparing the artificial lipid membranes over the microapertures of a microaperture array illustrating the embodiment of the present invention;
Fig. 3 is a sectional view showing a process of forming a lipid pattern around the microapertures of the microaperture array to prepare organic-solvent-free lipid membranes illustrating the embodiment of the present invention;
Fig. 4 is a diagram showing an aspect of peeling off a second Parylene film illustrating the embodiment of the present invention;
Fig. 5 is a diagram showing the prepared lipid pattern and its thickness illustrating the embodiment of the present invention;
Fig. 6 is a diagram showing the artificial lipid membranes and their uniformity prepared in a preliminary experiment according to the present invention; and
Fig. 7 shows confocal images of the dome-shaped artificial lipid membranes produced over the microapertures by an electroformation method illustrating the embodiment of the present invention.

### DESCRIPTION OF THE PREFERRED EMBODIMENTS

The method of preparing artificial lipid membranes over microapertures of a substrate includes forming a lipid pattern on the perimeter of microapertures formed in a substrate, swelling the lipid pattern by an electroformation method, fusing the swelled lipid pattern, and further swelling the fused lipid pattern for each microaperture to prepare dome-shaped organic-solvent-free lipid membranes.

### Embodiments

Hereinbelow, embodiments of the present invention will be described in detail.

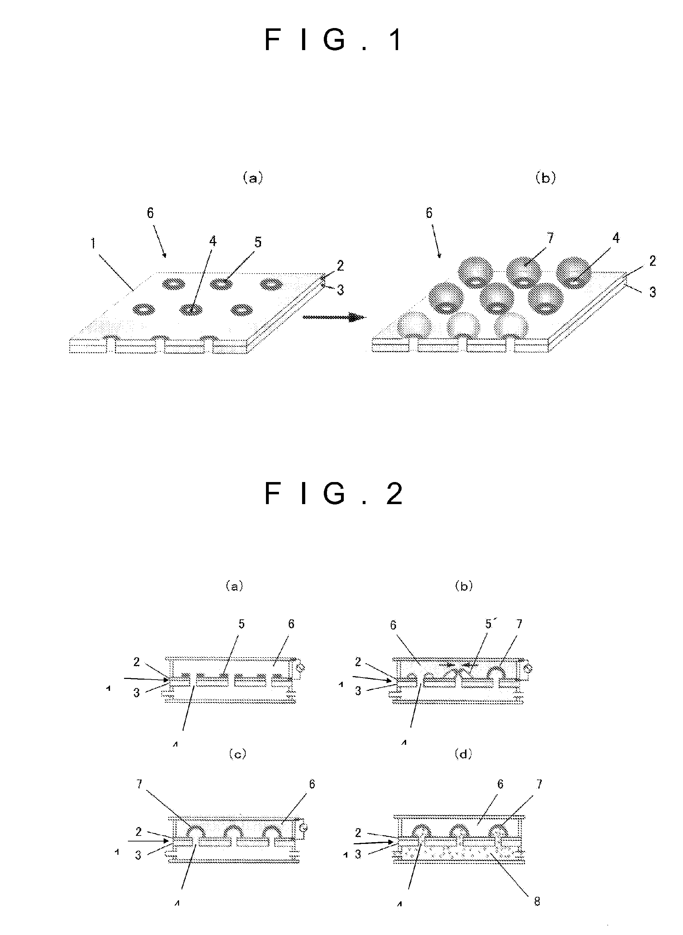

Fig. 1 is a perspective view showing a formation process of an artificial lipid membrane array having dome-shaped artificial lipid membranes over microapertures illustrating an embodiment of the present invention, and Fig. 2 is a sectional view showing a formation process of preparing the artificial lipid membranes over the microapertures of a microaperture array.

Hereinbelow, the dome-shaped artificial lipid membranes over the microapertures of the microaperture array and the formation of the artificial lipid membrane array will be described in detail.

As shown in Figs. 1 and 2(a), first, on a substrate 1 made of a Parylene film (Parylene sheet) 3 having a Cr/Au electrode film 2 on its surface, arrayed microapertures (e.g., micropores; 3 or 5 µm in diameter) 4 are formed, a doughnut-shaped lipid pattern (e.g., with the outer diameter of 10 or 15 µm) 5 is formed on the perimeter of the microapertures (micropores) 4 using the Parylene lift-off technique (see Non-Patent Document 7 above), and then the substrate 1 is immersed into a buffer (water) 6. Although the Cr/Au electrode film is preferred here as described above, other metals such as Al or ITO may be used as long as it is electrically conductive.

Next, as shown in Fig. 2(b), by using an electroformation method, i.e., by applying an AC electric field, the doughnut-shaped lipid pattern 5 is swelled and fused to form lipid membranes 5'.

By further swelling the fused lipid membranes 5', a lipid membrane 7 swelled into a dome shape is formed over each microaperture 4, as shown in Fig. 2(c).

The advantage of this method is, as shown in Fig. 2(d), that a solution 8 contained in the dome-shaped lipid membranes 7 can be exchanged through each microaperture 4 after the formation of the membranes.

In addition, the lipid membrane array having these lipid membranes 7 allows parallel measurements of the activity of ion channels. Moreover, using the doughnut-shaped lipid pattern 5 allows control of the size of the lipid membranes 7.

Although the electroformation method is introduced here as the preferred technique, the hydration method as a common method of producing liposomes may be used.

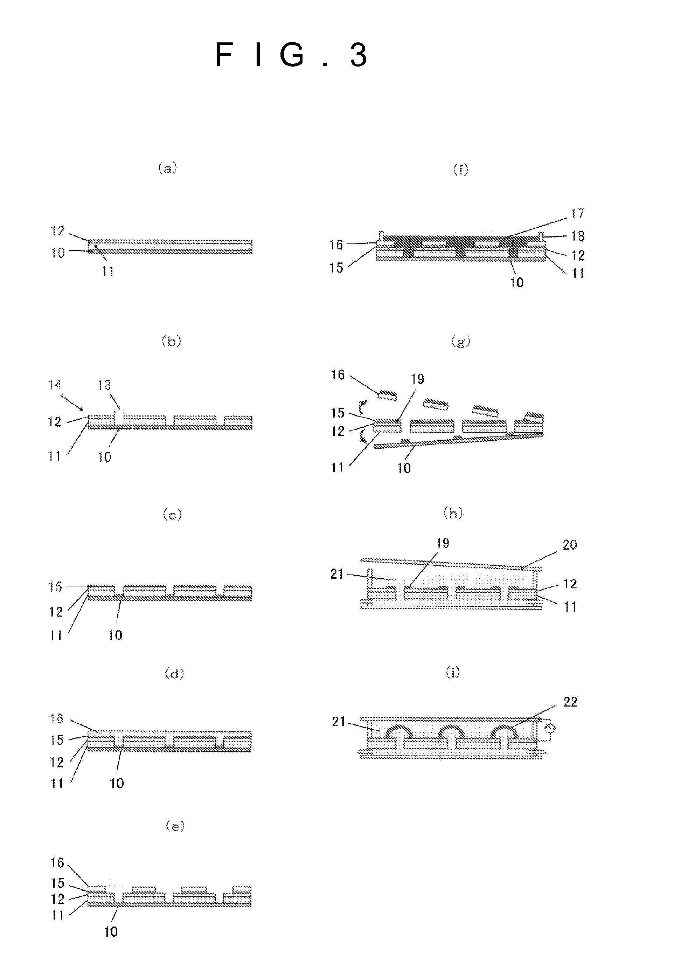

Fig. 3 is a sectional view showing a process of forming a lipid pattern around the microapertures of the microaperture array to form organic-solvent-free lipid membranes illustrating the embodiment of the present invention.

The Parylene film is used here because it can be easily fabricated into a diaphragm structure having microapertures by using standard photolithography and can be easily removed from the surface of a silicon substrate when the lipid pattern is formed on the Cr/Au electrode film.

(1) Make an electrode film having microapertures (micropores) 13.

First, on a silicon wafer (silicon substrate) 10, a first Parylene (C or N) film 11 with 5 µm in thickness, for example, was deposited using the CVD method. Then, a Cr/Au electrode film 12 was evaporated on the first Parylene film 11 (see Fig. 3(a)). Then, a photoresist was spun on the Cr/Au electrode film 12 and the first Parylene film 11, and standard lithography was performed. By applying O₂ plasma (15 ml/min, 50 W for 10 min), the first Parylene film 11 was etched, resulting in an array 14 having microapertures 13 (see Fig. 3(b)). Although the silicon substrate is preferred as a substrate, it is not limited thereto.

(2) Deposit a second Parylene film 16 having apertures for forming a lipid pattern on the Cr/Au electrode film 12 and the first Parylene film 11.

First, on the array 14 prepared in (1) above, an intermediate layer 15 was formed (see Fig. 3(c)). The second Parylene film 16 was deposited thereon and patterned in the similar manner as that for the first Parylene film 11 (see Fig. 3(d) and (e)). The thickness of the second Parylene film 16 was 4 µm, for example.

In this experiment, the second Parylene film 16 had to attach to the surface of the Cr/Au electrode film 12 when a phospholipid solution 17 is introduced, and had to be peeled off easily from an Au surface with a mechanical force during the lipid patterning. In order to meet these conditions, the intermediate layer 15 was deposited between the Au surface on the first Parylene film 11 and the second Parylene film 16. In order to find the ideal intermediate layer 15, Cytop (cyclized perfluoropolymer, Asahi Glass Co., Ltd., Japan), 0.2% detergent (micro-90, International Products Corp.), and a 1-octadecanethiol (ODT, CH₃-(CH₂)₁₇-SH) molecular film (Wako Pure Chemical Industries, Ltd., Japan) were explored. ODT forms a self-assembly monolayer on a gold surface. Regions of the intermediate layer 15 where the lipid pattern would be formed were removed simultaneously with the second Parylene film 16 being etched off by O₂ plasma (see Fig. 3(e)).

(3) Next, form the lipid pattern on the microapertures by peeling off the second Parylene film 16.

First, the phospholipid solution 17 was introduced into a silicone rubber chamber 18 and evaporated by supplying nitrogen gas (see Fig. 3(f)). Then, a doughnut-shaped lipid pattern 19 was formed by peeling off the second Parylene film 16 (see Fig. 3(g)). Then, the lipid pattern 19 was stored in a vacuum chamber for at least two hours to evaporate an organic solvent completely.

Used as the phospholipid solution 17 was egg yolk L-α-phosphatidyl choline (EPC) 1.5 mg/ml (Sigma-Aldrich Co.) and fluorescent DiI (1,1'-dihexadecyl-3,3,3',3'-tetramethylindocarbo-cyanieperchlorate, ex/em: 549/564 nm, Molecular Probes) diluted with methanol (Kanto Chemical Co., Inc., Japan).

(4) Form the organic-solvent-free lipid membranes over the microapertures using the electroformation method.

Here, an ITO (indium tin oxide) glass 20 was used as an electrode, and positioned away from the first Parylene film 11 having the lipid pattern 19 by a silicon spacer chamber filled with a degassed buffer (water) 21 (see Fig. 3(h)). When the AC electric field (10 Hz, 0.1-1.0 V_{PP}) was applied to the electrodes, organic-solvent-free dome-shaped lipid membranes 22 were formed around the microapertures.

In this manner, the lipid membrane array having the microapertures could be made successfully on the Cr/Au electrode film and the Parylene C or N film. The surface of the laminated Parylene N film is less rough than that of the Parylene C film. Therefore, the Parylene N film is suitable for use as the substrate for patterning the thin lipid membrane.

On the other hand, the effect of the intermediate layer on adhesion is as shown in Table 1. In Table 1, O denotes the condition of Fig. 4(a), and × denotes the condition of Fig. 4(b).

**[Table 1]**

| Intermediate layer | None | O₂ Plasma | Cytop | (0.2%) detergent | ODT |
|---|---|---|---|---|---|
| after second Parylene film lift-off | × | × | ○ | ○ | ○ |
| Comments | - | - | Long time process | May not suit for lipid patterning | Short time process (2 mins) |

As shown in Fig. 4(a), when peeling off the second Parylene film, the Cr/Au electrode film must remain on the first Parylene film, and as shown in Fig. 4(b), the Cr/Au electrode film must not be peeled off along with the second Parylene film.

The second Parylene film can be peeled off using tweezers in each case. However, as shown in Table 1, without the intermediate layer, the Cr/Au electrode film is also peeled off along with the second Parylene film.
On the other hand, when Cytop, the detergent, and ODT are used as the intermediate layer, the Cr/Au electrode film remains on the first Parylene film. ODT is deposited by the simplest process in these three types, and less affect on biomaterials as compared to Cytop and the detergent. Therefore, in this experiment, ODT was used as the intermediate layer for forming the lipid pattern.

As described above, by using the Parylene lift-off technique, the lipid pattern could be made easily around the microapertures on the Cr/Au electrode film, and the lipid membrane array could be made successfully.

Fig. 5 is a diagram showing the prepared lipid pattern and its thickness illustrating the embodiment of the present invention.

Fig. 5(a) and (b) show fluorescence images of the lipid pattern before and after peeling off the second Parylene film, respectively. It can be observed that the lipid pattern is stained with red color over the whole area before peeling off the second Parylene film. On the other hand, after peeling off the second Parylene film, only regions of the apertures of the second Parylene film are stained. In this manner, by using the lift-off technique of the Parylene film, the doughnut-shaped (with the outer diameter of 15 µm) lipid pattern is formed successfully around the microapertures (with the diameter of 5 µm) at a 30 µm interval, as shown in Fig. 5(c). Fig. 5(d) shows an AFM image and a profile of the thickness of the lipid pattern. The result of the cross-sectional analysis by the AFM reveals that the lipid thickness of 400 nm is obtained in the case where the concentration of the lipid is 1.5 mg/ml. The thickness can be controlled by the concentration of the phospholipid solution.

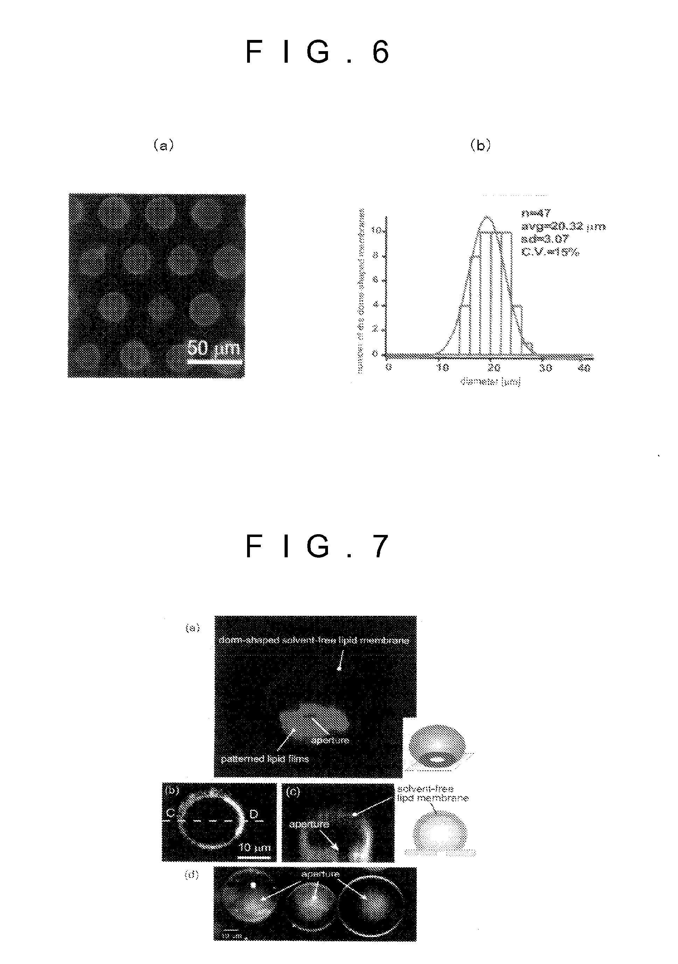

Fig. 6 is a diagram showing the artificial lipid membranes and their uniformity prepared in a preliminary experiment according to the present invention.

Here, the preliminary experiment was performed for making the lipid membranes from the lipid pattern on the ITO glass substrate without apertures using the electroformation method.

Fig. 6(a) is a fluorescence image of the lipid membranes prepared in this preliminary experiment. In this electroformation method, the AC electric field (10 Hz, 1.5 V) was applied for two hours. Fig. 6(b) shows the number and diameter of the prepared lipid membranes. Here, the coefficient of variation (C.V. = standard deviation (sd)/mean value (avg) × 100%) of the diameters of the prepared lipid membranes, i.e., the uniformity of the lipid membranes, was improved to 15% from 53% in the case without the lipid pattern. This result shows that the lipid pattern is useful for the formation of the lipid membranes in a narrow size distribution.

Fig. 7 shows confocal images of the dome-shaped lipid membranes prepared over the microapertures of the microaperture array by the electroformation method illustrating the embodiment of the present invention, wherein Fig. 7(a) shows a two-dimensional image at the XY axis, Fig. 7(b) shows a two-dimensional image at the XZ axis, Fig. 7(c) shows a three-dimensional image reconstructed from the cross-sectional image at the XY axis, and Fig. 7(d) shows the lipid membranes arranged in an array over the microapertures.

Here, as the instrument for observing the fluorescent images of the lipid pattern, a fluorescence microscope (IX-71, Olympus Corp., Japan) with a green excitation filter (WIG, Olympus Corp., Japan) was used. These fluorescent images were recorded by a 3CCD digital camera (C7780, Hamamatsu Photonics, Japan) and imaging software (AquaCosmos, Hamamatsu Photonics, Japan). In addition, a confocal microscopy system (CSU 22, Yokogawa Electric Corp., Japan; Cascade II, Photometrics; IX-70, Olympus Corp., Japan; 532 nm laser-Ventus, Laser Quantum Ltd.) was used to obtain the cross-sectional images of the prepared lipid membranes. Moreover, by using image processing software (MetaMorph, Molecular Devices, Inc.), two-dimensional side views and three-dimensional images of the produced lipid membranes were reconstructed from the cross-sectional confocal images. The thickness of the lipid pattern was measured by an atomic force microscope (AFM) (VN-8010, Keyence Corp., Japan).

As described above, the dome-shaped artificial lipid membranes can be obtained over the microapertures of the microaperture array by the electroformation method. In addition, the artificial lipid membranes can be formed into liposomes by freeing the dome-shaped artificial lipid membranes from the substrate.

Although the above-described embodiments have been described with regard to the microaperture array, it is not limited thereto and a single artificial lipid membrane can be prepared over a single microaperture. In addition, it may apply to a substrate for holding such a single artificial lipid membrane.

In addition, the present invention should not be limited to the embodiments described above, and a number of variations are possible on the basis of the spirit of the present invention. These variations should not be excluded from the scope of the present invention.

According to the present invention, the method of preparing the organic-solvent-free artificial lipid membranes over the microapertures of the substrate and the substrate for holding the artificial lipid membranes can be obtained.

### INDUSTRIAL APPLICABILITY

The lipid membranes prepared by the method of preparing the artificial lipid membranes over the microapertures of the microaperture array according to the present invention is widely applicable to membranes for analyzing proteins as the new usage.

## Claims

1. A method of preparing artificial lipid membranes over microapertures of a substrate, comprising the steps of:
(a) forming a lipid pattern on the perimeter of the microapertures formed in the substrate; and
(b) swelling the lipid pattern by an electroformation method, fusing the swelled lipid pattern, and further swelling the fused lipid pattern for each microaperture to prepare dome-shaped organic-solvent-free lipid membranes.

2. The method of preparing artificial lipid membranes over microapertures of a substrate according to claim 1, wherein the substrate is made of a Parylene film having an electrode film deposited thereon.

3. The method of preparing artificial lipid membranes over microapertures of a substrate according to claim 2, wherein the electrode film is a Cr/Au electrode film.

4. The method of preparing artificial lipid membranes over microapertures of a substrate according to claim 2, wherein the lipid pattern on the perimeter of the microapertures is formed by a Parylene lift-off technique.

5. The method of preparing artificial lipid membranes over microapertures of a substrate according to claim 4, wherein the lipid pattern on the perimeter of the microapertures is formed by depositing a first Parylene film on a bottom substrate, depositing the Cr/Au electrode film thereon, etching the first Parylene film and the Cr/Au electrode by lithography, then forming an intermediate layer on the Cr/Au electrode film, depositing a second Parylene film on the intermediate layer, etching the second Parylene film and the intermediate layer by the lithography, applying a lipid solution thereon, peeling off the second Parylene film and the bottom substrate, and storing in a vacuum chamber for a few hours.

6. The method of preparing artificial lipid membranes over microapertures of a substrate according to claim 5, wherein the intermediate layer is ODT.

7. The method of preparing artificial lipid membranes over microapertures of a substrate according to claim 1, wherein the dome-shaped organic-solvent-free lipid membranes are prepared by disposing the substrate with the lipid pattern formed on the perimeter of the microapertures in a chamber containing a buffer introduced therein, and using the electroformation method.

8. A substrate for holding artificial lipid membranes comprising dome-shaped organic-solvent-free artificial lipid membranes formed over microapertures of a substrate having a film with an electrode film deposited thereon, that is peeled off easily.

9. The substrate for holding artificial lipid membranes according to claim 8, wherein the film that is peeled off easily is made of a Parylene film.
